# EUROPEAN PATENT APPLICATION

(11) **EP 2 510 781 A1**
(43) Date of publication of application: **17.10.2012**
(21) Application number: 11305457.1
(22) Date of filing: 15.04.2011
(51) Int. Cl.: A01H 5/08, C12N 15/82

(54) **Novel methods of modifying plant phenotype**

(71) Applicant: INSTITUT NATIONAL DE LA RECHERCHE AGRONOMIQUE, 75007 Paris (FR)
(72) Inventor: Bendahmane, Mohammed, 69700 Chassagny (FR); Raymond, Olivier, 69006 Lyon (FR); Vergne, Philippe, 69007 Lyon (FR)
(74) Representative: Becker, Philippe

(57) **Abstract**

The present invention relates to methods for generating plants having modified phenotypes, and to plants so generated and parts of these plants. In particular, the present invention relates to novel methods for producing non-transgenic plants with a desired phenotype via transmission of the RNA-mediated transcriptional gene regulation signal from the rootstock to the scion. The invention also relates to methods of inhibiting or activating expression of target genes in an engrafted plant via RNA-mediated transcriptional gene regulation in a non coding region of a target gene. The invention also relates to rootstocks altered via RNA-mediated transcriptional regulation, and to a use of the altered rootstocks for inducing the gene silencing or gene up-regulation in scions and for modifying the phenotype of engrafted plants.

SEQUENCE LISTING

<110>  INRA

<120>  Novel methods of modifying plant phenotype

<130>  B1174EP00

<160>  6

<170>  PatentIn version 3.3

<210>  1
<211>  452
<212>  DNA
<213>  Arabidopsis thaliana

<400>  1
gggaaatggt acaaagttaa aggagatctg agtgagagtt agataataaa tgaaaagaaa      60

taagaaacca tcagggtttt ttctaatgtg gagttttaga ttcagttttg tagaactaag     120

attcactttg ttgggtgttc tttcttcact catttctgtt attataataa taataaaatc     180

ttatatcttt ctattttcct tactaacaag tacttgaaga tttagatata tttatagatc     240

tggtgttgta ataggtaaaa acttgatttt tatgactata aaagtaagtt ttgggaaaca     300

aattggggag agagtaagga aggactatga ggtcatatct tctgttttgt gatcatccat     360

cctccattgt tgttaatgtc tgtgtctctc tttttcttct cttctttctc ttactttcct     420

ttcttatctc tagctctctt tctctctcat ga                                   452


<210>  2
<211>  24
<212>  DNA
<213>  artificial sequence

<220>
<223>  forward primer

<400>  2
gggaaatggt acaaagttaa agga                                             24


<210>  3
<211>  26
<212>  DNA
<213>  artificial sequence

<220>
<223>  reverse primer

<400>  3
tcatgagaga gaaagagagc tagaga                                           26

<210>  4
<211>  280
<212>  DNA
<213>  Rosa sp.

<400>  4
caatttctta gctcttggtt tggtagaagc atagatctgg tgtaaaacat ggaaaaagaa      60

ttatacaagg gatggaaata gaaactagta gtgggttttg gaatgtatgc aggcctctga     120

gctcagctcc ttgccttcct ccattgctgt taatttttct gtttcctttc tctctttttt     180

agtttctctt tctctctcac tcgatcactc tctgcaacac tctatgtcat ctatgaaggc     240

ctacaacaca agctgtcatg ctcctattag cccagtcccc                           280


<210>  5
<211>  23
<212>  DNA
<213>  artificial sequence

<220>
<223>  left primer

<400>  5
caatttctta gctcttggtt tgg                                              23


<210>  6
<211>  20
<212>  DNA
<213>  artificial sequence

<220>
<223>  right primer

<400>  6
ggggactggg ctaataggag                                                  20

## Description

### FIELD OF THE INVENTION

The invention relates generally to the field of botanic and agriculture. More specifically, the present invention relates to methods for generating plants having modified phenotypes, and to plants so generated and parts of these plants. In particular, the present invention relates to novel methods for producing non-transgenic plants with a desired phenotype via transmission of the RNA-mediated transcriptional gene regulation signal from the rootstock to the scion. The invention also relates to methods of inhibiting or activating expression of target genes in an engrafted plant via RNA-mediated transcriptional gene regulation in a non coding region of a target gene. The invention also relates to rootstocks altered via RNA-mediated transcriptional regulation, and to a use of the altered rootstocks for inducing the gene silencing or gene up-regulation in scions and for modifying the phenotype of engrafted plants.

### BACKGROUND OF THE INVENTION

The genetic transformation of plants and the modulation of expression of certain plant genes are important tools for generating modified plants which exhibit altered phenotypes having commercially useful properties.

Numerous techniques are available for the transformation of a number of plant species by altering the gene expression at the level of DNA or at the level of RNA.

Recombinant DNA technology is one of the most important strategies to produce genetically modified plant cells. With recombinant DNA technology, it is possible, for example, to cut a fragment of DNA from a donor species, introduce it into the DNA of another host species, with which the donor species cannot cross, but in which the donor DNA is expressed. The genetically modified host species, thus acquires a new property, which can't by obtained by conventional breeding techniques.

During the last years, thousands of genetic sequences of genomic or cDNA origin have been indentified and cloned from hundreds of plant species. With the recent development of advanced rapid techniques for cloning and sequencing and the completion or near-completion of the sequencing of entire genomes, such as for example for Arabidopsis and rice, attention has been re-focused on to the characterization of isolated sequences and the elucidation of their in vivo function. One approach for carrying out these studies involves the production of a range of mutant plant lines exhibiting loss-of-function or gain-of-function phenotypes, and the subsequent selection and characterization of the mutant phenotypes produced.

The expression of genetic sequences may also be modulated by gene silencing using RNA interference, ribozyme or antisense technologies, based on the introduction of sense and/or antisense copies of particular genetic sequences.

Gene silencing has been particularly studied over the past decades for production of plants of interest by inhibiting the expression of target genes. It has been reported that the silencing does not depend on the presence of endogenously encoded homologous loci (Tijsterman et *al.,* 2002). Moreover, silencing can occur between two related transgenes, and it is not only triggered by transgenes but can also be initiated by viruses known as VIGS for virus induced gene silencing (Angell et *al.,* 1997).

Further experiments with transgenic plants have revealed the existence of two different mechanisms of gene silencing which can be mediated at either the transcriptional level or at the post-transcriptional level (Vaucheret et al., 2006, Genes & Dev 20: 759-771).

The first mechanism of gene silencing at the transcriptional level (TGS or transcriptional gene silencing) involves alterations at the DNA level and prevents transcription. TGS occurs mainly when multiple repeats of a transgene are inserted in the genome of transgenic plants. It correlates with condensation of chromatin and with DNA methylation.

The second mechanism of gene silencing at the post-transcriptional level (PTGS or post-transcriptional gene silencing or RNA silencing) involves mRNA degradation. It has been proposed that PTGS is triggered by double-stranded RNA and requires a conserved set of gene products (Matzke et *al.,* 2001). The mechanism for RNA silencing also involves short RNA species of around 25 nucleotides, corresponding to both sense and antisense sequence of the targeted mRNA (Hamilton and Baulcombe, 1999). These species, designated siRNAs, were found in a broad spectrum of plant systems undergoing PTGS, including transgene and virus-induced silencing. It has also been reported that transgenes are much better targets for PTGS than are endogenous genes, and that transcriptionally active genes are better inducers of PTGS than transcriptionally inactive genes (Mlotshwa et *al.,* 2002; Vaucheret et *al.,* 1997; Palauqui et *al.,* 1997).

The outcome of transcriptional and post-transcriptional gene silencing is a reduction in the accumulation of gene transcripts. Both types of silencing seem to be related since they are correlated with DNA methylation: in promoter regions in TGS and in transcribed or coding regions in PTGS (Mlotshwa et *al.,* 2002). However, the role of DNA methylation, especially in PTGS processes remains unclear.

In conclusion, genetic modifications of plants might involve insertion, deletion or substitution of various plant genes using numerous techniques, such as e.g., site-specific mutagenesis, ethyl methanesulfonate mutagenesis, targeting induced local lesions in genomes (TILLING), homologous recombination, conjugation, knock-out techniques, etc. Plant genes may also be inactivated by gene silencing induced by RNA interference.

A particular approach may be gene overexpression by insertion a DNA sequence through transposon mutagenesis using mobile genetic elements called transposons.

Other methods exploit natural forms of gene transfer, such as the ability of *Agrobacterium* to transfer genetic material to plants.

However, techniques of producing modified plants by genetic engineering, have many disadvantages since the transformation process of certain plants is very long. Moreover, modified plants obtained with genetic engineering techniques are considered as genetically modified organisms (GMO), the generation and the use of which is very strictly regulated since they might be associated with a range of issues presenting risks to human health or the environment.

Consequently, there exists a high demand for novel improved methods for modifying plant phenotypes and producing plants of interest.

### SUMMARY OF THE INVENTION

The present invention provides novel and efficient methods for producing non-transgenic plants with a desired phenotype.

More particularly, the invention provides a new method of modifying the phenotype of an engrafted plant by inhibiting or activating the expression of a target gene via RNA-mediated transcriptional target gene regulation.

Surprisingly, the inventors have discovered that transcriptional gene regulation can be transmitted from rootstocks to scions upon grafting. To our knowledge, this is the first example of stable long distance transmission of transcriptional gene regulation (such as silencing or activating a target gene) in engrafted plants.

A first object of this invention therefore relates to a method of modifying the phenotype of an engrafted plant, comprising:
a) providing a rootstock, wherein expression of a target gene in said rootstock is altered via RNA-mediated transcriptional regulation in a non coding region of said gene; and
b) grafting on said rootstock of a scion to form an engrafted plant, said grafting causing transmission of the RNA-mediated transcriptional gene regulation from the rootstock to the scion, thereby modifying the phenotype of the engrafted plant.

In this regard, expression of a target gene may be inhibited or activated.

Another object of the invention relates to a method of modifying the phenotype of an engrafted plant, comprising:
a) providing a rootstock, wherein a target gene in said rootstock is silenced via RNA-mediated transcriptional gene silencing (TGS) or is activated by RNA-mediated gene derepression in a non coding region of said gene; and
b) grafting on said rootstock of a scion to form an engrafted plant, said grafting causing transmission of the RNA-mediated transcriptional gene silencing (TGS) or transmission of the RNA-mediated gene derepression from the rootstock to the scion, thereby modifying the phenotype of the engrafted plant.

Another object of the invention relates to a method of modifying the phenotype of an engrafted plant, comprising:
a) providing a rootstock, wherein a target gene in said rootstock is silenced via RNA-mediated transcriptional gene silencing (TGS) in a non coding region of said gene; and
b) grafting on said silenced rootstock of a scion to form an engrafted plant, said grafting causing transmission of the RNA-mediated transcriptional gene silencing (TGS) from the rootstock to the scion, thereby modifying the phenotype of the engrafted plant.

Another object of the invention relates to a method of modifying the phenotype of an engrafted plant, comprising:
a) providing a rootstock, wherein a target gene in said rootstock is activated by RNA-mediated gene derepression in a non coding region of said gene; and
b) grafting on said silenced rootstock of a scion to form an engrafted plant, said grafting causing transmission of the RNA-mediated gene derepression from the rootstock to the scion, thereby modifying the phenotype of the engrafted plant.

Another object of the invention relates to a method of inhibiting expression of a target gene in an engrafted non-transgenic plant, comprising:
a) providing a rootstock, wherein a target gene in said rootstock is silenced via RNA-mediated transcriptional gene silencing (TGS) in a non coding region of said gene; and
b) grafting on said silenced rootstock of a scion to form an engrafted plant, said grafting causing transmission of the RNA-mediated transcriptional gene silencing (TGS) from the rootstock to the scion, thereby inhibiting expression of the target gene in the engrafted plant.

Another object of the invention relates to a method of activating expression of a target gene in an engrafted plant, comprising:
a) providing a rootstock, wherein a target gene in said rootstock is activated via RNA-mediated transcriptional gene derepression by blocking a repressor site in a non coding region of said gene, and
b) grafting on said rootstock of a scion to form an engrafted plant, said grafting causing transmission of the RNA-mediated gene derepression from the rootstock to the scion, thereby activating the expression of the target gene in an engrafted plant.

In a particular embodiment, the methods of the invention comprise transforming the rootstock with a DNA molecule expressing an RNA molecule that inhibits or activates the expression of the target gene.

The RNA molecule is preferably a single-stranded RNA (ssRNA), double-stranded RNA (dsRNA), short interfering RNA (siRNA), small activating (saRNA), micro RNA (miRNA), small heterochromatic RNA (shRNA), or a hairpin RNA (hpRNA) molecule. Preferably, said RNA molecule induces cytosine methylation, histone modification or chromatin modification. Most preferably, the RNA molecule is a siRNA or a saRNA.

In a preferred embodiment, the RNA is a RNA molecule that causes selective methylation of the target gene in the selected non-coding region thereof.

The invention also relates to a use of an RNA molecule that inhibits the expression of a target gene, for modifying the phenotype of an engrafted plant via transcriptional gene silencing (TGS) transmitted from a rootstock.

The invention also relates to a use of an RNA molecule that activates the expression of a target gene, for modifying the phenotype of an engrafted plant via transcriptional gene derepression transmitted from a rootstock.

Another object of the invention relates to an RNA molecule that inhibits the expression of AGAMOUS, PLOK or TCTP gene in the engrafted plant via transcriptional gene silencing.

Another object of the invention relates to a rootstock, wherein expression of a target gene in said rootstock is altered via RNA-mediated transcriptional regulation in a non coding region of said gene, and wherein said regulation is transmissible to a scion grafted on the rootstock.

Another object of the invention relates to a rootstock, wherein a target gene in said rootstock is silenced via RNA-mediated transcriptional gene silencing (TGS) in a non coding region of said gene, for inducing the RNA-mediated TGS of said gene in a scion grafted on the rootstock.

Another object of the invention relates to a rootstock, wherein a target gene in said rootstock is activated via RNA-mediated gene derepression by targeting a transcriptional repressor in a non coding region of said gene, for inducing the RNA-mediated gene derepression of said gene in a scion grafted on the rootstock.

The invention also relates to plants comprising or derived from the above described rootstock, to seeds of such plants and to plants or descendents of plants grown or derived from said seeds.

The invention is applicable to any plant and target gene. In particular, the target gene may be selected from genes involved in the regulation of meiosis, pollen production, flower development, number of petals, replacing stamens by petals, modifying organ size (e.g. reducing total size so as to obtain miniature plants), or altering secondary metabolites synthesis so as to modify color (by disrupting the production of a pigment) or to change scent (by disrupting the production of a volatile compound). The invention is applicable to produce vegetables, fruits and ornamental plants having desired phenotypes.

### BRIEF DESCRIPTION OF THE DRAWINGS

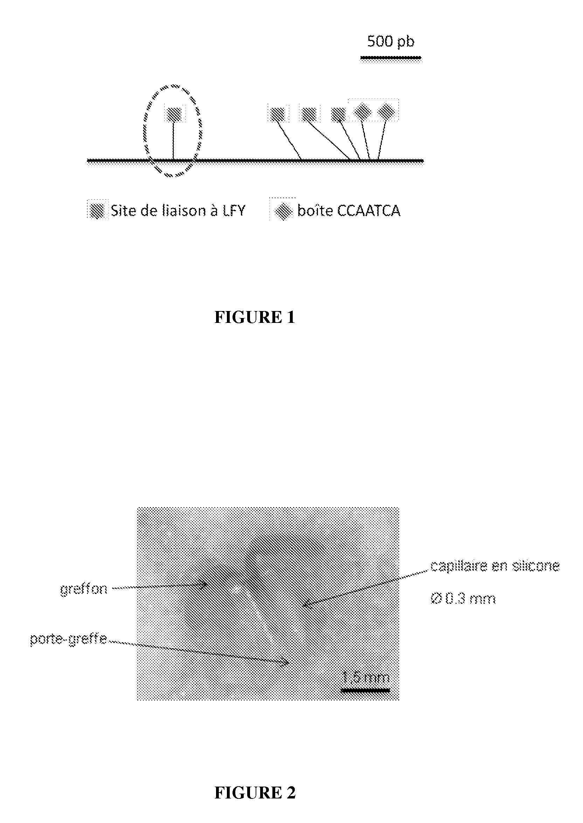

**Figure 1****:** Structure of second intron of *AGAMOUS* gene at *Arabidopsis thaliana* with its principal regulating elements. Site of binding to LFY region by an RNAi is surrounded.
**Figure 2****:** Device used for grafting of a scion of *Arabidopsis thaliana* on a rootstock according to the invention. The hypocotyls of the scion and of the rootstock are maintained in contact one with the other by a silicone capillary.

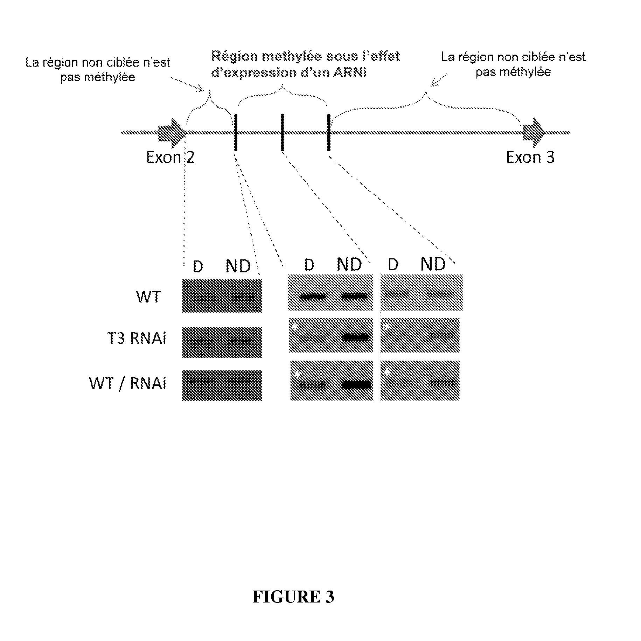

**Figure 3****:** Analysis of methylation in the region of the second intron of *AGAMOUS* gene of *Arabidopsis thaliana* by digestion of the genomic DNA with McrBC enzyme followed by PCR. McrBC acts only on methylated sites: after PCR, the methylated areas are less amplified than DNA controls which were not digested. The genetic transformation of *A. thaliana* (Columbia ecotype) was carried out with p35S:: TGS-AG by *Agrobacterium tumefaciens.* The selection of T1 plants was carried out on medium ms + kanamycine. The resistant seedlings were put onto compost without selection, and then transferred into culture chamber until flowering. D: Digested DNA; ND: control not digested DNA; * shows methylation of the region targeted by RNAi, which is sensitive to digestion by McrBC enzyme. WT: genomic DNA of a wildtype *A. thaliana;* T3 RNAi: genomic DNA of a line expressing RNAi targeting the region of the second intron 2 of AGAMOUS gene of *Arabidopsis thaliana;* WT/RNAi: genomic DNA of a wildtype scion (not transformed) on the RNAi-silenced rootstock.

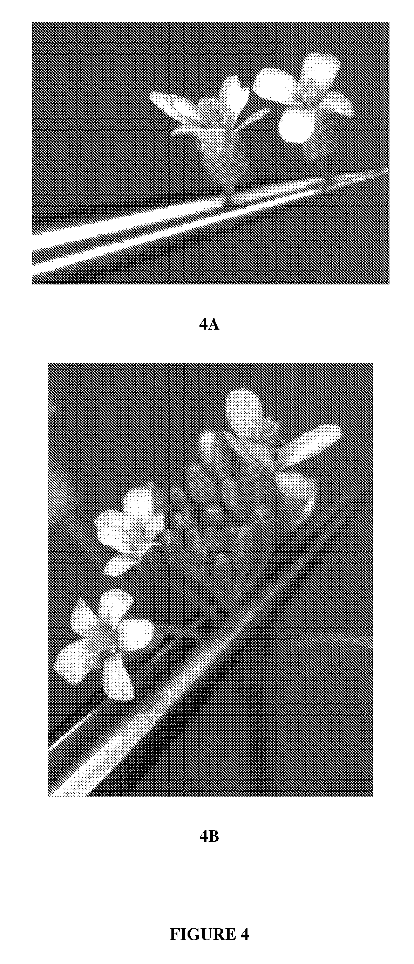

**Figure 4****:** Modification of the phenotype of *Arabidopsis thaliana* plant obtained by the method of the invention. **4A** presents a photo of two flowers: a flower of modified *Arabidopsis thaliana* line (i.e., AG-TGS line obtained via transmission of TGS of *AGAMOUS* gene according to the method of the invention), and a wild line flower of *Arabidopsis thaliana.* The AG-TGS flower has increased number of petals in comparison to a flower of a wild line of *Arabidopsis thaliana* having only 4 petals; **4B** presents a photo of an inflorescence of the modified AG-TGS line showing a cluster of flowers arranged on a stem; all the flowers of the modified plant present increased number of petals, with several variations.

### DETAILED DESCRIPTION OF THE INVENTION

The present invention relates to novel methods of generating plants with a desired phenotype. In particular, the inventors have found a way to modify the phenotype of an engrafted plant without modifying the genome of said plant. More specifically, the modification is made in a rootstock and is then stably transmitted to the plant by grafting.

Palauqui et *al.,* 1997 have reported that post-transcriptional silencing (PTGS) of the *uidA* transgene encoding glucuronidase, can be transmitted by grafting from silenced transgenic tobacco plants to non-silenced plants expressing the transgene. However, PTGS requires the presence of a transgene in the target scion and the transgene must be transcriptionally active for the scion to be competent for silencing.

Mlotshwa et *al.,* 2002, have recently reported that the dsRNA-induced transcriptional gene silencing (TGS) produced in the *H_{9NP}* and *271* tobacco lines, is not transmissible from rootstocks to scions. Grafting experiments demonstrate that only post-transcriptional gene silencing (PTGS) is graft transmissible. Moreover, the authors underline the unability of TGS to move systemically and they conclude that TGS is not graft-transmissible.

Furthermore, international application WO 2007/033437 relative to a method of inhibiting of a target gene via transcriptional and post-transcriptional gene silencing, clearly indicates that RNA silencing (PTGS) is systematically transmitted from the transgenic rootstock to the target scion, whereas cytosine methylation of the transgene is not.

Surprisingly, the inventors have now discovered that transcriptional gene silencing (TGS) can be graft-transmissible. More specifically, the present application shows that TGS can be efficiently transmitted from a silenced rootstock to a non-silenced scion by grafting. This is illustrated in the experimental part by detection of the same methylation pattern of a target DNA in the rootstock and in the scion thus confirming the transmission of DNA methylation from the rootstock to the scion. The results obtained by the inventors also illustrate plants with modified phenotype having flowers with increased number of petals.

The inventors have also discovered that expression of a target gene may be activated by RNA-mediated transcriptional gene regulation, and that transcriptional gene activation can also be graft-transmissible.

To our knowledge, this is the first example of a method of obtaining engrafted plants with a desired phenotype, which is based on transmission of RNA-mediated transcriptional gene regulation from the rootstock to the scion, such a gene regulation being transcriptional gene down-regulation or gene up-regulation. Preferably, the transcriptional gene down-regulation is a transcriptional gene silencing (TGS), and the transcriptional gene up-regulation is preferably a gene derepression.

In this regard, it is noted that TGS pathway is very advantageous in comparison with PTGS pathway.

TGS pathway takes place upstream, at the transcriptional level and, is much more efficient and stable than PTGS pathway since TGS allows to amplify the silencing signal in comparison with PTGS targeting mRNA which undergoes numerous modifications and/or degradation (e.g. polyadenylation, splicing etc.) resulting in a reduced silencing signal.

Moreover, PTGS is essentially active for silencing transgenes, not endogenous genes and, additionally, in PTGS, target genes must be transcriptionally active (Mlotshwa et al., 2002).

Furthermore, TGS is meiotically heritable, and it correlates with DNA modification manifested by hypermethylation of promoters of silenced genes or with changes of chromatin structure. Contrary to TGS, PTGS is not meiotically transmitted and must be reestablished in each generation.

The present invention provides, for the first time, a method of stable graft-transmissible RNA-mediated transcriptional gene silencing (TGS). In particular, the invention provides a method of preparing rootstocks with silenced target genes and using these rootstocks to induce transcriptional target gene silencing in scions grafted on the rootstocks.

In addition, the inventors have also demonstrated that transmission of RNA-mediated transcriptional gene silencing, from rootstocks to scions, involves RNA-directed DNA methylation in the engrafted plant. Such a mechanism of transmission at the transcriptional level indicates that RNA molecules involved in DNA methylation of a target gene in the engrafted plant must be transmitted to the cell nuclei of the scion in order to induce DNA methylation and inactivate the target gene.

Thus, the inventors have demonstrated, for the first time, the potential of transcriptional gene silencing to mediate DNA methylation in cell nuclei of the engrafted plants, thus demonstrating long distance transmission of transcriptional gene silencing. The inventors have also confirmed that transmission of DNA methylation in silenced scions is maintained and stable.

The present invention resides in the ability to transmit RNA-mediated transcriptional target gene signaling (causing gene down-regulation or gene up-regulation) in the engrafted plants and thus modify their phenotype without modifying the genotype of commercialized plants.

The present disclosure will be best understood by reference to the following definitions:

### Definitions

The method of the present invention may be applied to any plant which can be propagated by grafting or by cuttings. Within the context of the present invention, the engrafted plants include woody plants. In the most preferred embodiment, the engrafted plant is of the Rosaceae family. As used therein, the term "Rosaceae" designates all of the species and genera of the Rosaceae family. The Rosaceae family is a large family of flowering plants, with about 3000 species in 95 genera. The Rosaceae family comprises herbs, trees, shrubs and climbing plants. In particular, the Rosaceae according to the present invention, comprises edible fruits (such as apples, pears, plums, cherries, almonds, peaches, apricots, raspberries and strawberries) as well as ornamental trees and shrubs flowers (e.g., roses, meadowsweets, photinias, firethorns, rowans and hawthorns).

Within the context of the present invention, the term "target gene" designates any gene of interest of any plant that may be multiplied by grafting. In particular, a "target gene" of the invention includes a gene, the silencing of which induces a desired modification of the phenotype of the engrafted plant. Preferably, the "target gene" is selected from genes involved in meiosis, in pollen production, in flower development, in control of organ size, in metabolic pathways leading to the synthesis of pigments, volatile compounds or molecules contributing to the organoleptic properties of edible fruits.

Specific examples of target genes include, without limitation, *AGAMOUS, PLOK* or *TCTP.* Further examples of target genes are listed below:
- genes involved in floral development and architecture : *SEPALLATA, LEAFY, SHATTERPROOF* ; some of these genes might also play a part in the control of fruit shape and size ; some of them might also be involved in the maturation of pericarp and its evolution into a fleshy tissue ;
- genes involved in the synthesis of pigments and co-pigments : *CHALCONE ISOMERASE, CHALCONE SYNTHASE, DIHYDROFLAVONOL REDUCTASE, ANTHOCYANIDIN SYNTHASE, FLAVONOL SYNTHASE, FLAVONOL 3' HYDROXYLASE, FLAVONOL 3', 4' HYDROXYLASE;*
- genes involved in the synthesis of volatile compounds : *ORCINOL O-METHYLTRANSFERASE, EUGENOL SYNTHASE* (eugenol has allergenic properties and its removal may be desirable).

Typically, the target genes are endogenous gene.

Different embodiments of the present invention will now be further described in more details. Each embodiment so defined may be combined with any other embodiment or embodiments unless otherwise indicated. In particular, any feature indicated as being preferred or advantageous may be combined with any other feature or features indicated as being preferred or advantageous.

### Rootstock production and its use for modifying the phenotype of an engrafted plant

As described above, the present invention is based on the use of an altered rootstock as inducer of a desired phenotype in the engrafted plant via RNA-mediated transcriptional regulation a target gene. The inventors have surprisingly discovered that RNA-mediated transcriptional gene down- or up-regulation may be transmissible to a scion grafted on the rootstock.

More specifically, the present invention provides a method of modifying the phenotype of an engrafted plant, comprising (i) providing a rootstock, wherein a target gene in said rootstock is silenced via RNA-mediated transcriptional gene silencing (TGS) or is activated via RNA-mediated gene derepression in a non coding region of said gene; and (ii) grafting on said rootstock of a scion to form an engrafted plant, said grafting causing transmission of the RNA-mediated transcriptional gene silencing (TGS) or gene derepression from the rootstock to the scion, thereby modifying the phenotype of the engrafted plant.

Rootstocks of the present invention are typically prepared by (i) selection of a target gene, (ii) identification of a non-coding region within said target gene, (iii) preparing a nucleic acid construct allowing the expression of a transcriptional silencing or activating signal via RNA interference specific for said region of said gene, and (iv) preparing a transgenic rootstock containing and expressing said nucleic acid construct.

As indicated above, the target gene may be any gene of interest, preferably any gene the regulation of which impacts on the phenotype of a plant. Examples of target genes include, without limitation, genes involved in meiosis, in pollen production, in flower development, in control of organ size, in metabolic pathways leading to the synthesis of pigments, volatile compounds or molecules contributing to the organoleptic properties of edible fruits, for instance.

The success of the invention derives, inter alia, from the discovery that transcriptional gene regulation may be transmissible and stable if the target gene regulation occurs in a non-coding region thereof. Such non-coding region may be, for instance, a promoter region sequence, a regulatory sequence, a repressor site sequence, a terminator sequence, or an intron sequence. For each candidate target gene, a proper region may be identified, depending on the type of regulation needed (i.e., up or down). For instance, to obtain silencing of a gene, an activating region within a promoter sequence may be selected. In the contrary, to produce activation of target gene expression, a repressor site in a promoter may be selected, to cause de-repression. Given a particular target gene, it is possible, by specifically acting on non-coding regions that activate or repress the expression of the gene to obtain either its down-regulation or its up-regulation. It is therefore possible, according to the method of the invention, to manipulate target gene expression through the transmission of a regulatory signal from a transformed rootstock to a non-GMO scion in order to obtain plants with desirable traits.

Once a non coding region of a target gene has been selected, a nucleic acid construct allowing RNA-mediated transcriptional regulation of said target gene should be prepared. Such a construct shall typically encode an RNA molecule that is specific for said region and functions as a gene silencing or activating signal. Specific examples of such RNA molecules include single-stranded RNA (ssRNA), double-stranded RNA (dsRNA), small interfering RNA (siRNA), small activating (saRNA), micro RNA (miRNA), short heterochromatic RNA (shRNA), or hairpin RNA (hpRNA) molecules.

To produce TGS, it is preferred to engineer a nucleic acid construct encoding interfering RNA species, particularly siRNAs or hpRNas.

To produce TGA, it is preferred to engineer a nucleic acid construct encoding activating RNA species, particularly saRNAs or hpRNAs.

The active RNAs typically comprise at least a sequence that is complementary to a portion of the non-coding region of the target gene. Depending on their nature, these RNAs may be single- or double-stranded, contain loop(s) or hairpin structure(s).

The transcriptional gene regulation according to the present invention is essentially caused or induced by RNA-mediated DNA methylation (e.g., de novo methylation), induction of histone modification and/or induction of chromatin modification. The inventors have indeed confirmed that expression of a nucleic acid construct causing DNA methylation at a specific non-coding region of a gene causes regulation thereof, and that said regulation is stable and transmissible to a grafted plant.

Preferably, DNA methylation is or includes cytosine methylation in the non-coding sequence of the target gene.

In a particular embodiment, transcriptional gene silencing is induced by methylation or hypermethylation of key sites such as promoter or intronic regions of target genes. In this particular embodiment, the modification of gene expression occurs in the scion, through transcriptional deregulation by methylation of a promoter or intronic region of a target gene, leading to silencing or activation of the target gene. When methylation is targeted to binding sites of transcriptional repressors, overexpression of the target gene is caused.

A particular advantage of the method of the invention is that, being stable in time, the transformation (either silencing or derepression) obtained after methylation is expected to be maintained after the scion is removed from the transgenic rootstock and cultivated per se after the establishment of its own root apparatus. Furthermore, the invention does not require genetic modification of the genome of the resulting plant. Indeed, the nucleic acid construct introduced into the rootstock is not transmitted to the plant, but only the regulation signal.

As shown in the experimental part, the inventors have demonstrated that DNA methylation is transmissible from a rootstock to a scion since the regulatory sequence of the target *AGAMOUS* gene in the non-altered scion is methylated in the same way as the corresponding sequence of *AGAMOUS* gene the altered rootstock.

Furthermore, the inventors have also demonstrated that engrafted plants obtained by the method of the invention via transmission of the RNA-mediated transcriptional gene silencing of *AGAMOUS* gene, from the rootstock to the scion, present a modified phenotype since their flowers have increased number of petals.

The nucleic acid construct that allows expression of the RNA-mediated gene regulation according to the invention may be prepared according to methods known per se in the art, once the target gene region has been selected. Typically, the corresponding region is cloned in the reverse orientation, in one or several copies (e.g., tandem or head-to-head) under control of a functional promoter, the expression thereof leading to the production of functional and gene-specific RNA species. In a preferred embodiment, the construct causes production of hairpin RNA structures comprising a strand comprising a sequence specific of a non coding nucleic acid region of the target gene. In a most preferred embodiment, the nucleic acid construct comprises multiple repeats of the cloned region.

The nucleic acid construct is typically cloned (or part of) a vector compatible for cloning and/or expression into plant cells. In a specific embodiment, the nucleic acid construct and vectors are prepared using the Gateway technology for generating RNA hairpin structures containing a sequence complementary to a selected cDNA sequence. Using Gateway technology, the sequence targeted for gene silencing or gene derepression, is first cloned in an entry vector which is then recombined with a destination vector carrying two independent Gateway cassettes separated by an intron spacer, and allowing two copies of the target nucleic acid sequence to be positioned head to head in the resulting hpRNA expression clone.

Other methods of producing RNA mediated gene silencing/activation, which may be adapted for use in the present invention, are known per se in the art.

In a specific embodiment, a recombinant vector of the invention contains repeated inversion regions of an area of 452 pairs of bases (SEQ ID NO: 1) encompassing the first site of binding to LFY on the intron of *AGAMOUS* (see Figure 1). Preferably, such a recombinant vector utilizes CaMV35S promoter and contains the construct p35S:: TGS-AG.

In another specific embodiment, a recombinant vector contains a sequence specific of non coding nucleic acid sequence comprised in the sequence of 280bp (SEQ ID NO: 4) corresponding to a site LBS/WBS on the intronic region of *Rosa chinensis cv. Old Blush.*

To produce transgenic rootstocks of the invention, the nucleic acid construct or vector may be introduced into a plant cell by any means, including transfection, transformation, transduction, electroporation, particle bombardment, agroinfection, etc.

In a preferred embodiment, a nucleic acid molecule is introduced via Agrobacterium transformation using the Ti plasmid as described e.g., by Toki et al. (2006).

Generation of a rootstock from modified cells can be obtained using methods known per se to the skilled worker. In particular, it is possible to induce, from callus cultures or other undifferentiated cell biomasses, the formation of shoots and roots. The plantlets thus obtained can be planted out and used for cultivation. Methods for regenerating plants from cells are described, for example, by Fennell et al. (1992) Plant Cell Rep. 11: 567-570; Stoeger et al (1995) Plant Cell Rep. 14: 273-278; Jahne et al. (1994) Theor. Appl. Genet. 89: 525-533.

The invention also relates to constructs (e.g., nucleic acids, vectors, cells, etc) suitable for production of such altered rootstocks.

The present invention also relates to a recombinant vector comprising a DNA sequence expressing an RNA molecule that inhibits or activates the expression of the target gene as described above. Such a recombinant vector may be used for transforming a cell or a plant in order to inhibit or activate the expression of the target gene. Suitable vectors can be constructed, containing appropriate regulatory sequences, including promoter sequences, terminator fragments, polyadenylation sequences, enhancer sequences, marker genes and other sequences as appropriate. Preferably the nucleic acid in the vector is under the control of, and operably linked to an appropriate promoter or other regulatory elements for transcription in a host cell such as a microbial, (e.g. bacterial), or plant cell. The vector may be a bi-functional expression vector which functions in multiple hosts. In a preferred aspect, the promoter is a constitutive or inducible promoter.

Preferably, the expression vectors are Gateway overexpression vectors. In a preferred embodiment, the expression vector is pK7GWIWG2D(2).

In specific embodiments, the expression vector contains a sequence of SEQ ID NO: 1 which is specific of AtAGAMOUS, or a sequence of SEQ ID NO: 4 which is specific of RoAGAMOUS. Typically, the expressed molecule adopts a hairpin conformation and stimulates generation of RNA molecules against the non-coding sequence.

In the most preferred embodiment, the rootstocks of the invention comprises a nucleic acid sequence expressing an RNA molecule that inhibits or activates the expression of a target gene at the transcriptional level. Preferably, such a transcriptional gene silencing or activating is associated with cytosine methylation. In particular embodiments, such rootstocks can produce RNA molecules that inhibit or activate the expression of AGAMOUS gene, PLOK gene or TCTP gene via cytosine methylation.

In another embodiment, the invention also relates to regulatory nucleic acid, such as an RNA molecule that induces the transcriptional silencing or activation of a target gene. (e.g., AGAMOUS, PLOK or TCTP).

More specifically, the invention relates to an RNA molecule that inhibits or activates the expression of a target gene in the engrafted plant via transcriptional gene silencing (TGS) or via transcriptional gene derepression transmitted from an alteredrootstock to a non altered scion by means of grafting.

In particular, the invention also relates to the use of such a regulatory nucleic acid or RNA molecule for modifying the plant phenotype.

The present invention also relates to altered rootstocks for grafting and for inducing the transcriptional target gene silencing or activation in the engrafted plant. Such rootstocks are obtained by methods of the present invention as described above. In a particular embodiment, such rootstocks contain an inactive target gene which was previously silenced via RNA-mediated transcriptional gene silencing (TGS) in a non coding region. In another particular embodiment, such rootstocks contain an active target gene which was previously activated via RNA-mediated transcriptional gene derepression in a non coding region.

Examples of such rootstocks for grafting and for inducing the gene silencing, include rootstocks with a silenced gene selected from the group consisting of AGAMOUS, PLOK or TCTP.

In a particular embodiment, the invention relates to the rootstock for inducing the silencing of AGAMOUS gene. Preferably, the silencing of AGAMOUS gene in the engrafted plant modifies the number of flower petals.

In another particular embodiment, the invention relates to the rootstock for inducing the silencing of PLOK gene. Preferably, the silencing of PLOK gene in the engrafted plant increases the frequency of diploid gametes.

In another particular embodiment, the invention relates to the rootstock for inducing the silencing of TCTP gene. Preferably, the silencing of TCTP gene in the engrafted plant enables generation of a dwarf plant.

A further particular object of this invention relates to plants comprising or derived from the rootstock as described above.

A further object of this invention relates to seeds of plants of the invention, or to plants, or descendents of plants grown or otherwise derived from said seeds.

The invention also relates to vegetal material of a plant of the invention, such as roots, leaves, flowers, callus, etc.

### Grafting

Grafting may be performed according to techniques known per se in the art. In a preferred embodiment the grafting step is carried out as described in Example 2 below. Surprisingly, the inventors have shown that such a grafting allows transmission of silencing signal from the silenced rootstock, as described above, to a distant non-silenced scion, thus modifying in a targeted and stable way the phenotype of an engrafted plant.

### Applications of the method of the invention

As described above, the method of the invention combines (i) the step of preparing a rootstock, wherein a target gene is silenced via RNA-mediated transcriptional gene silencing (TGS) in a non coding region with the step of (ii) long distance transmitting of the RNA-mediated TGS in the engrafted plant via grafting of an non-silenced scion on the silenced rootstock. Such a combination allows to modify the phenotype of the engrafted plant by inhibiting expression of a target gene, said inhibition of the expression of a target gene (i.e., silencing of the target gene) inducing the phenotype of interest.

The ability to modify the phenotype of a plant may be useful, for example, for producing plants with more highly desired characteristics, such as delayed flowering, increased lateral branching, delayed senescence etc. In particular embodiments, the method of the invention can be used to produce vegetables, fruits and ornamental plants with desired characteristics, e.g., edible plants with increased quantity of fruits, vegetables and fruits of bigger size, ornamental plants with increased number of petals or having specific color, etc. In a particular embodiment, the method of the invention allows to change the phenotype of fruits, so as to provide fruits which will have improved aspects for storage, handling, cooking, organoleptic properties, freezing, nutritional value, etc.

Examples of plants with modified phenotype according to the invention are listed below:
- ornamental plants with flowers harboring specific patterns of volatile compounds synthesis, e.g., plants devoid of eugenol which has allergenic properties;
- ornamental plants with flowers harboring modified pigment synthesis, e.g., plants with increased synthesis of paeonidin 3-monoglucoside, an anthocyanin giving specific hue to red flower tones;
- cut-flowers with increased vase-life;
- plants with edible fruits with modified pericarp lignifications, e.g., raspberries without pit inside the druplets, or strawberries with soft akenes

Further examples of such plants are disclosed in the experimental section.

In a particular embodiment, the method of the invention is carried out for producing engrafted plants with a silenced AGAMOUS gene. Preferably, the method of the invention is carried out for producing flowering plants with controlled number of flower petals (for example in wild species of ornamental plants such as roses, e.g., *Rosa moyesii* or *Rosa canina).*

In another particular embodiment, the number of petals of plants obtained according to the method of the invention is increased in comparison with the number of petals of wildtype plants.

In another particular embodiment, the method of the invention allows to generate the engrafted plants with increased frequency of diploid gametes in diploid scions.

In another particular embodiment, the method of the invention is carried out for producing engrafted plants with a silenced PLOK gene, for modifying the phenotype of plants in association, for example, with the control of male meiosis or with the production of non reduced pollen.

In another embodiment, the pollen of the engrafted plants with a silenced PLOK may be used for crossing with tetraploid varieties in order to obtain tetraploid progeny starting from the first generation, with no need to pass by triploid generations, which are not fertile enough.

In another particular embodiment, the method of the invention is carried out for producing engrafted plants with increased resistance to various pathogens.

In a further embodiment, the method of the invention is carried out for producing engrafted dwarf plants. Preferably, such dwarf plants are obtained by using transgenic rootstock of the invention with a silenced TCTP gene, which enables creation of dwarf plants.

Further aspects and advantages of the invention are provided in the following examples, which are given for purposes of illustration and not by way of limitation.

### EXAMPLES

### EXAMPLE 1: Preparation of silenced rootstocks from R. chinensis

### Transformation protocol

### Production and maintenance of embryogenic calli

In vitro propagated shoots of *R. chinensis* cv Old Blush (or *A. thaliana)* are used as starting material. They are repeatedly sub-cultured every 4 weeks on proliferation medium, PM (Murashige and Skoog [MS] salts and vitamins with 0.1 g 1⁻¹ Fe-EDDHA, 30 g 1⁻¹ sucrose, 0.02 µM a-naphthaleneacetic acid [NAA], 0.29 µM gibberellic acid A3 [GA3], 4.44 µM 6-benzyladenine [BA]) *(Dohm et al.* 2001a), solidified by 2.4 g 1⁻¹ Gelrite™ (Duchefa Biochemie, Haarlem, The Netherlands) instead of agar. Mature leaves are injured by several cuts across their leaflet midbrids and plated with their adaxial side down onto callus induction medium, CIM (MS salts and vitamins, 30 g 1⁻¹ sucrose, solidified by 2.4 g 1⁻¹ Gelrite™, containing 2.69, 5.37 (Dohm et al. 2001a), 8.06 or 10.74 (this work) µM NAA). Thirty leaves are used for each NAA concentration (six leaves per petri dish). For each of the four NAA concentrations, resulting calli are then isolated from leaf remnants, pooled and squeezed with forceps onto paper filters layered on the embryo induction medium, EIM (MS salts and vitamins, 30 g 1⁻¹ sucrose, 18.24 µM zeatin, 2.4 g 1⁻¹ Gelrite™) *(Dohm et al.* 2001a). Three dishes are prepared for each NAA concentration experiment.

Embryo forming callus are transferred for proliferation to embryogenic callus maintenance medium, EMM (MS salts and vitamins, 30 g 1⁻¹ sucrose, 1.34 µM NAA, 6.84 µM zeatin and 3 µM GA3, solidified by 2.4 g 1⁻¹ Gelrite™) (Dohm *et al.* 2001a). Calli are then subcultured onto fresh EMM medium every 4-6 weeks: parts showing vitrification or necrosis are removed, selecting for embryogenic tissues.

Except for shoot micropropagation and rooting, all experiments are performed in 100 x 20 mm Petri dishes containing 34 ml medium. Dishes are sealed with Parafilm™. Unless otherwise stated, all experiments are performed at 25°C in light conditions (16 h day/8 h night, 20-40 µMol m-2 s-1, provided by Osram Biolux tubes [Osram GmbH, Regensburg, Germany]) in Sanyo incubators (Environmental test chamber MLR-350H, Sanyo Electric co., Ltd, Moriguchi, Osaka, Japan).

### Vectors and Agrobacterium tumefaciens mediated transformation

Transformation is essentially performed as described (Dohm *et al.* 2001b; Vergne *et al.,* 2010), with modifications. EHA105 *Agrobacterium tumefaciens* strain (Hood *et al.* 1993) harboring a hairpin construction in pK7GWIWG2D(II), is used as a vector to transform somatic embryos.

Any promoter or intronic region can be targeted in order to drive transcriptional gene silencing.

For *AtAGAMOUS (AtAG),* we targeted the intronic region of 452 bp of SEQ ID NO: 1.

For *RoAGAMOUS* (RoAG)*,* we targeted the intronic region of 280 bp of SEQ ID NO: 4.

Using Gateway technology (Invitrogen), the PCR amplification fragment was cloned using primers of SEQ ID NO: 2 and 3 (for *A.thaliana*) or primers of SEQ ID NO: 5 and 6 (for *R. chinensis*) as an inverted repetition into the vector : pK7GWIWG2D(II) (Karimi *et al.,* 2002 ; http://gateway.psb.ugent.be/vector/show/pK7GWIWG2D%28II% 29/search/index/).

Bacteria are plated onto solid YEP medium (Sambrook *et al.* 1989) supplemented with rifampicin and kanamycin (50 mg 1⁻¹ each) for 48-72 h at 28°C. One isolated colony is inoculated in four ml liquid YEP medium (kanamycin 50 mg 1⁻¹) and cultured for 24 h at 28°C and 200 rpm, until OD is about 1.8. Two ml of this preculture are diluted in 40 ml of a minimal medium (MinA, Temmerman *et al.* 2000) supplemented with glucose (2 g 1⁻¹) and cultured for 2 h in the dark at 28°C and 100 rpm to initiate Vir gene expression.

Somatic embryos (100 clusters of 2-5 embryos), are dissected from 4-6 weeks old embryogenic cultures and plated on a filter paper on EMM, for 24-48 h. Embryos are then bulk wounded with sterile washed sea sand twice for 2 min at 450 rpm and rinsed with MinA to remove sand. They are then co-cultivated in bacterial culture for 1 h at 60 rpm at room temperature. They are dried on filter paper and plated on a shoot inducing medium (SIM: MS salts and vitamins, 30 g 1⁻¹ sucrose, 0.05 µM indole-3-butyric acid [IBA], 8.9 µM BA and 0.3 µM GA3, solidified with 7.2 g 1⁻¹ agar [Microbiological Agar-Agar, Merck, Darmstadt, Germany]) without selection for 2 days in the dark. Embryos are then washed several times (1 min, 250 rpm) with 30 ml water containing cefotaxime sodium (500 mg 1⁻¹) and carbenicillin (50 mg 1⁻¹) to eliminate bacteria in excess and dried onto filter paper before being plated on SIM medium containing cefotaxime sodium (500 mg 1⁻¹) and carbenicillin (50 mg 1⁻¹). About 10 days after transformation, embryos are transferred onto EMM supplemented with kanamycin (30 mg 1⁻¹) and Timentin™ (150 mg 1⁻¹), and subcultured every 3-4 weeks, to promote formation of transformed embryos. Newly formed transformed embryos are then isolated and plated onto SIM supplemented with 30 mg 1⁻¹ kanamycin for adventitious shoot organogenesis. After 4-6 weeks, regenerated shoots were transferred to selective proliferation medium (with 2.22 µM BA and 7.2 g 1⁻¹ agar [Microbiological Agar-Agar, Merck, Darmstadt, Germany] instead of 4.44 µM BA and Gelrite™, respectively) supplemented with 30 mg 1⁻¹ kanamycin. Starting from this stage the antibiotics are omitted in the following subcultures.

Regenerated shoots are transferred to proliferation medium (with 2.22 1M BA and 7.2 g 1⁻¹ agar [Microbiological Agar-Agar, Merck, Darmstadt, Germany] instead of 4.44 1M BA and Gelrite™, respectively) for further development. In the first subculture, proliferation medium is supplemented with 30 mg 1⁻¹ kanamycin, while the antibiotic is omitted in the subsequent subcultures.

### Root induction and greenhouse acclimatization of transformed plants

Well differentiated plantlets originating from transformed embryos are transferred onto rooting medium containing Indole-3-acetic acid (IAA) (MS salts at half strength and vitamins, 30 g 1-1 sucrose, 11.4 µM IAA, solidified by 2.4 g 1⁻¹ Gelrite™). Vessels are incubated for 2 days in the dark at 22°C and then transferred to light conditions at the same temperature for 4 weeks. Rooted plantlets (with at least two roots longer than 2 cm) are carefully pulled out of medium and placed into soil under a shaded polyethylene tent at 25°C and 90% relative humidity. After 2-3 weeks, plants are then acclimatized in a confined greenhouse following standard procedures: pots are covered with polyethylene bags which were gradually opened over a period of 3 weeks.

### EXAMPLE 2: Grafting procedure

The grafting protocol has been carried out as shown in Figure 2. Grafting was performed following standard procedure known as T-budding or shield budding, which is a special grafting technique in which the scion piece is reduced to a single bud. The plant propagated (using the bud) is referred to as the scion, while the plant being grafted onto is referred to as the rootstock. The whole of the steps is detailed below:

### Scion preparation :

Buds are retrieved from stems bearing senescent flowers. In order to assess if the stem is suitable for buds harvest, one evaluation criterion is that epidermal thorns should be easy to remove. Buds for grafting should be prepared so that no medullar parenchyma remains.

### Rootstock preparation :

Rootstock are prepared from cuttings of a plant (e.g., *Rosa chinensis* or *Arabidopsis thaliana*)*.* Cuttings (5 cm long stems for *Rosa chinensis* for example) are rooted on fertiss substrate at 28°C under 90% hygrometry. Rooting usually occurs within three to four weeks of culture. Afterward, rooted cuttings are transferred to pots (dimension: 6 x 6 x 7.2 cm) containing standard substrate and grown for three to four weeks under standard greenhouse conditions at 25°C. Finally, rooted cuttings are transferred to one liter pots. A T-incision is cut in the epidermis with a scalpel so that the underlying cambium is not injured. The position of incision must be underneath the newly developed stems of the cuttings.

### Grafting :

Harvested buds are carefully inserted into the T-incision. Margins of the incision are then wrapped using Parafilm. All newly developed stems must be removed periodically from the rootstock to facilitate the scion growth.

### EXAMPLE 3: Silencing of AGAMOUS gene in via transmission of Transcriptional Gene Silencing (TGS) from the rootstock to the scion

The rootstock of *Arabidopsis thaliana* was prepared according to transformation protocol as described in Example 1.

Target intronic sequence for the TGS of AGAMOUS in *Arabidopsis thaliana* is of total legth of 452bp. This sequence is the following (SEQ ID NO: 1):

GGGAAATGGTACAAAGTTAAAGGAGATCTGAGTGAGAGTTAGATAATAAATGAAAA
GAAATAAGAAACCATCAGGGTTTTTCTAATGTGGAGTTTTAGATTCAGTTTTGTAGA
ACTAAGATTCACTTTGTTGGGTGTTCTTTCTTCACTCATTTCTGTTATTATAATAATAAT
AAAATCTTATATCTTTCTATTTTCCTTACTAACAAGTACTTGAAGATTTAGATATATTTAT
AGATCTGGTGTTGTAATAGGTAAAAACTTGATTTTTATGACTATAAAAGTAAGTTTTG
GGAAACAAATTGGGGAGAGAGTAAGGAAGGACTATGAGGTCATATCTTCTGTTTTGT
GATCATCCATCCTCCATTGTTGTTAATGTCTGTGTCTCTCTTTTTCTTCTCTTCTTTCTC
TTACTTTCCTTTCTTATCTCTAGCTCTCTTTCTCTCTCATGA

The primers used for PCR amplification of the above region were the following:
Forward primer : GGGAAATGGTACAAAGTTAAAGGA (SEQ ID NO: 2)
Reverse primer: TCATGAGAGAGAAAGAGAGCTAGAGA (SEQ ID NO: 3)

### Results:

As shown in Figure 3, TGS signal was transmitted from the transgenic rootstock to a non-transgenic scion.

Analysis of methylation of the second intron of *AGAMOUS* gene of *Arabidopsis thaliana* by digestion of the genomic DNA with McrBC enzyme (which acts only on methylated sites) followed by PCR show that methylation of the region targeted by RNAi is found in both: (i) genomic DNA of a line expressing RNAᵢ targeting the region of intron 2, analyzed in the present study (T3 RNAi); and in (ii) genomic DNA of a wildtype scion (not transformed) on the RNAᵢ-silenced rootstock (WT/RNAᵢ) (see Figure 3).

In addition, no methylation was detected in the regions which were not targeted by RNAᵢ.

These results demonstrate, for the first time, that DNA methylation of target regions is transmissible from the rootstock to the scion, thus confirming that TGS signal has been transmitted from the silenced rootstock to the non-silenced scion.

Moreover, the inventors have demonstrated that engrafted plants obtained by the method of the invention have flowers with increased number of petals as shown in Figures 4A and 4B.

### EXAMPLE 4: RNA-mediated activation of AGAMOUS gene in Rosa chinensis via methylation of a repressor site in a non coding region

The rootstock of *Rosa chinensis* was prepared according to transformation protocol as described in Example 1.

### Caracterization of the target region:

The targeted region belongs to an intron regulating the expression of *AGAMOUS* in *Rosa* species.

The sequence of this region as well as the sequence of primers used for the construction of the transgene inducing the production of RNAᵢ molecules is given below for *Rosa chinensis* cv. Old Blush.

Thus, the targeted region is the following (SEQ ID NO: 4):

CAATTTCTTAGCTCTTGGTTTGGTAGAAGCATAGATCTGGTGTAAAACATGGAAAAA
GAATTATACAAGGGATGGAAATAGAAACTAGTAGTGGGTTTTGGAATGTATGCAGGC
CTCTGAGCTCAGCTCCTTGCCTTCCTCCATTGCTGTTAATTTTTCTGTTTCCTTTCTCT
CTTTTTTAGTTTCTCTTTCTCTCTCACTCGATCACTCTCTGCAACACTCTATGTCATCTA
TGAAGGCCTACAACACAAGCTGTCATGCTCCTATTAGCCCAGTCCCC

PRODUCT SIZE: 280, PAIR ANY COMPL: 5.00, PAIR 3' COMPL: 1.00

TARGETS (start, len)*: 40,187 (SEQ ID NO: 4)

The following primers were used for RNAᵢ TGS of the LBS/WBS site of *RoAGAMOUS,* based on intronic sequence of *Rosa chinensis* cv 'Old Blush'. They amplify a region of 280 bp (SEQ ID NO: 4) which is very well conserved in Rosaceae family. 
OLIGO start len tm gc% any 3' seq
LEFT PRIMER 12 23 59.34 39.13 4.00 0.00 CAATTTCTTAGCTCTTGGTTTGG
(SEQ ID NO: 5)
RIGHT PRIMER 291 20 59.92 60.00 5.00 2.00 GGGGACTGGGCTAATAGGAG (SEQ ID NO: 6)

It was described before that the methylation of the homologous intronic region in model plant *Arabidopsis thaliana* is associated to an alteration of floral phenotype, with a loss of the reproductive organs and floral reiteration (Jacobsen *et al.,* 2000).

However, when the intronic region (as described above) is targeted in *Rosa chinensis* cv. *Old Blush* by RNA interference according to the method of the invention, the obtained phenotype presents, on the contrary, a reduction of petals number. That means that the mechanism altered by RNA interference implies a repressor of the extinction of *AGAMOUS* gene.

### Results:

The phenotype of *Rosa chinensis* cv. *Old Blush* with a reduced petals number, which is obtained by the inventors shows that using RNA interference may activate the expression of the target gene *AGAMOUS* via methylation of a repressor of the target gene in a non coding region thus leading to the gene derepression.

### REFERENCES

Angell S.M., Baulcombe D.C. (1997) Consistent gene silencing in transgenic plants expressing a replicating potato virus X RNA. EMBO J. 16:3675-84
Dohm A, Ludwig C, Nehring K, Debener T (2001a) Somatic embryogenesis in roses. Acta Hort 547:341-347
Dohm A, Ludwig C, Schilling D, Debener T (2001b) Transformation of roses with genes for antifungal proteins. Acta Hort 547:27-33.
Hamilton A.J., Baulcombe D.C. (1999) A species of small antisense RNA in posttranscriptional gene silencing in plants. Science 286, 950-952
Hood EE, Gelvin SB, Melchers LS, Hoekema A (1993) New Agrobacterium helper plasmids for gene transfer to plants. Transgen Res 2:208-21
Jacobsen SE, Sakai H, Finnegan EJ, Cao X and Meyerowitz E. 2000. Ectopic hypermethylation of flower-specific genes in Arabidopsis. Current Biology 10 : 179-186.
Karimi M, Inzé D, Depicker A (2002) Gateway vectors for Agrobacterium-mediated plant transformation. Trends Plant Sci May;7(5): 193195
Matzke M., Matzke A.J., Kooter J.M. (2001) RNA: Guiding gene silencing. Science 293, 1080-1083.
Mlotswa S., Voinnet O., Mette M.F., Matzke M., Vaucheret H., Ding S.W., Pruss G., Vance V.B. (2002) RNA silencing and the mobile silencing signal. Plant cell: S289-S301
Palauqui J.C., Elmayan T., Pollien J.M., Vaucheret H. (1997) Systemic acquired silencing : transgene-specific post-transcriptional silencing is transmitted by grafting from silenced stocks to non-silenced scions. EMBO J. No. 15 pp.4738-4745
Sambrook J, Fritsch EF, Maniatis T (1989) Molecular cloning: a laboratory manual, 2nd edn. Cold Spring Harbor Laboratory Press, Plainview
Temmerman W, Vereecke D, Dreesen R, Van Montagu M, Holsters M, Goethals K (2000) Leafy gall formation is controlled by fasR, an AraC-type regulatory gene in Rhodococcus fascians. J Bact 182:5832-5840
Tijsterman M., Ketting R.F., Plasterk H.A. (2002° The genetics of RNA silencing. Annu. Rev. Genet. 36:489-519
Vaucheret H., Nussaume L., Palauqui J.C., Quillere I., Elmayan T. (1997) A transcriptionally active state is required for post-transcriptional silencing (co-suppression) of nitrate reductase host genes and transgenes. Plant Cell 9:1495-504
Vergne P, Maene M, Gabant G, Chauvet A, Debener T, Bendahmane M (2010) Somatic embryogenesis and genetic tranformation of the diploid Rosa chinensis cv Old Blush. Plant Cell Tiss Organ Cult 100:73-81

## Claims

1. A method of modifying the phenotype of an engrafted plant, said method comprising the following steps:
a) providing a rootstock, wherein expression of a target gene in said rootstock is altered via RNA-mediated transcriptional regulation in a non coding region of said gene; and
b) grafting on said rootstock of a scion to form an engrafted plant, said grafting causing transmission of the RNA-mediated transcriptional gene regulation from the rootstock to the scion, thereby modifying the phenotype of the engrafted plant.

2. The method according to claim 1, wherein expression of the target gene is inhibited or activated.

3. The method of modifying the phenotype of an engrafted plant according to claim 1 or 2, said method comprising the following steps:
a) providing a rootstock, wherein a target gene in said rootstock is silenced via RNA-mediated transcriptional gene silencing (TGS) or is activated by RNA-mediated gene derepression in a non coding region of said gene; and
b) grafting on said rootstock of a scion to form an engrafted plant, said grafting causing transmission of the RNA-mediated transcriptional gene silencing (TGS) or transmission of the RNA-mediated gene derepression from the rootstock to the scion, thereby modifying the phenotype of the engrafted plant.

4. A method of inhibiting expression of a target gene in an engrafted plant, said method comprising:
a) providing a rootstock, wherein a target gene in said rootstock is silenced via RNA-mediated transcriptional gene silencing (TGS) in a non coding region of said gene; and
b) grafting on said rootstock of a scion to form an engrafted plant, said grafting causing transmission of the RNA-mediated transcriptional gene silencing (TGS) from the rootstock to the scion, thereby inhibiting expression of the target gene in the engrafted plant.

5. A method of activating expression of a target gene in an engrafted plant, said method comprising the following steps:
a) providing a rootstock, wherein a target gene in said rootstock is activated via RNA-mediated transcriptional gene derepression by blocking a repressor site in a non coding region of said gene, and
b) grafting on said rootstock of a scion to form an engrafted plant, said grafting causing transmission of the RNA-mediated gene derepression from the rootstock to the scion, thereby activating the expression of the target gene in an engrafted plant.

6. The method according to anyone of the preceding claims, wherein said transcriptional gene regulation is mediated by RNA molecules selected from single-stranded RNA (ssRNA), double-stranded RNA (dsRNA), small interfering RNA (siRNA), small activating (saRNA), micro RNA (miRNA), short heterochromatic RNA (shRNA) or hairpin RNA (hpRNA) molecules.

7. The method according to claim 6, wherein said RNA molecules induce cytosine methylation, histone modification or chromatin modification in the target gene in the engrafted plant.

8. The method according to anyone of the preceding claims, wherein said rootstock is transformed with a DNA molecule expressing said RNA molecule.

9. The method according to claim 8, wherein the scion does not comprise said DNA molecule.

10. The method according to anyone of the preceding claims, wherein the target gene is involved in regulation of meiosis, increasing pollen production, flower development, increasing a number of petals, replacing stamens by petals, volatile compounds synthesis, pigment synthesis, modifying of fruit pericarp lignifications.

11. The method according to anyone of the preceding claims, wherein the target gene is selected from the group consisting of AGAMOUS, PLOK or TCTP.

12. The method according to anyone of the preceding claims, wherein the plant is from the family of Rosaceae.

13. An RNA molecule that alters the expression of AGAMOUS, PLOK or TCTP gene via transcriptional gene silencing.

14. The RNA molecule of claim 13 that binds to AGAMOUS non coding sequence, said RNA molecule having a sequence complementary to one or more fragments of the sequence of SEQ ID NO: 1 or 4.

15. The RNA molecule of claim 13 or 14 consisting of 19 to 25 nucleotides in length, preferably 24 nucleotides.

16. Use of an RNA molecule that inhibits or activates the expression of a target gene, for modifying the phenotype of an engrafted plant via transcriptional regulation transmitted from a rootstock.

17. A rootstock, wherein expression of a target gene in said rootstock is altered via RNA-mediated transcriptional regulation in a non coding region of said gene, and wherein said regulation is transmissible to a scion grafted on the rootstock.

18. The rootstock according to claim 17, wherein a target gene in said rootstock is silenced via RNA-mediated transcriptional gene silencing (TGS) in a non coding region of said gene, for inducing the RNA-mediated TGS of said gene in a scion grafted on the rootstock, or wherein a target gene in said rootstock is activated via RNA-mediated gene derepression by targeting a transcriptional repressor in a non coding region of said gene, for inducing the RNA-mediated gene derepression of said gene in a scion grafted on the rootstock.

19. The rootstock according to claim 17 or 18, wherein said rootstock comprises a DNA sequence expressing an RNA molecule that inhibits or activates the expression of a target gene.

20. The rootstock according to claim 18 or 19, wherein said silenced gene is selected from the group consisting of AGAMOUS, PLOK or TCTP.

21. The rootstock according to claim 20, wherein AGAMOUS gene is silenced and wherein said rootstock induces a modification of the number of flower petals in the engrafted plant.

22. The rootstock according to claim 20, wherein PLOK gene is silenced and wherein said rootstock induces an increase of the frequency of diploid gametes in the engrafted plant.

23. The rootstock according to claim20, wherein TCTP gene is silenced and wherein said rootstock enables creation of dwarf plants.

24. A plant comprising or derived from a rootstock of anyone of claims 18 to 23.

25. A seed of the plant of claim 24.

26. A plant or a descendent of a plant grown or derived from the seed of claim 25.

27. An engrafted plant comprising or derived from a rootstock of anyone of claims 17 to 22.

28. The plant according to anyone of claims 24, 26 or 27, wherein the plant is from the family of Rosaceae.
